# EUROPEAN PATENT APPLICATION

(11) **EP 4 737 580 A1**
(43) Date of publication of application: **06.05.2026**
(21) Application number: 24849598.8
(22) Date of filing: 31.07.2024
(51) Int. Cl.: C12P 7/6427, C12N 9/02, C12N 15/70

(54) **METHOD FOR PRODUCING, IN BIOREACTOR, RESOLVIN D5 FROM DOCOSAHEXAENOIC ACID-ENRICHED OIL BY USING LIPOXYGENASE**

(30) Priority: 31.07.2023 KR 20230099914
(71) Applicant: Konkuk University Industrial Cooperation Corp, Seoul 05029 (KR)
(72) Inventor: OH, Deok-Kun, Gwacheon-si, Gyeonggi-do 13802 (KR); LEE, Jin, Seoul 05013 (KR)
(74) Representative: Cabinet Becker et Associés
(86) International application number: PCT/KR2024/011207
(87) International publication number: WO 2025/029031

(57) **Abstract**

The present invention relates to a composition and a method for producing resolvin D5, an endogenous substance in the human body, by means of a whole-cell reaction in flasks and bioreactors using a double 15-lipoxygenase derived from the bacterium *Archangium violaceum,* with polyunsaturated fatty acids obtained by hydrolysis and subsequent solvent extraction from a low-cost docosahexaenoic acid-enriched oil and high-purity docosahexaenoic acid reagents as substrates. More particularly, the present invention provides a method for obtaining docosahexaenoic acid at a purity of 90%, in which a docosahexaenoic acid-enriched oil is effectively hydrolyzed into polyunsaturated fatty acids comprising docosahexaenoic acid using a commercial lipase and an adsorption resin, and the resulting hydrolysate is then purified by solvent extraction, as well as a composition for use in such a method and a purified hydrolysate obtained thereby. In addition, the present invention provides a method for large-scale production of resolvin D5, an endogenous substance in the human body, in flasks and bioreactors through a whole-cell reaction using a double arachidonate 15-lipoxygenase derived from the bacterium *Archangium violaceum,* with a high-purity docosahexaenoic acid reagent as a substrate, as well as a composition for producing resolvin D5. According to the present invention, resolvin D5 can be produced in an environmentally friendly manner with high productivity and high yield, and thus can be usefully applied in various industrial fields, including pharmaceuticals, foods, and cosmetics.

## Description

### TECHNICAL FIELD

The present invention relates to a composition for producing high-purity resolvin D5 (RvD5) by a whole-cell reaction of a recombinant microorganism comprising a double-oxygenating arachidonate (ARA) 15-lipoxygenase (15-LOX) derived from *Archangium violaceum* in a bioreactor, and to a method for producing RvD5, an endogenous substance in the human body, using a hydrolysis extract of a docosahexaenoic acid (DHA)-containing oil as a substrate derived from a low-cost DHA-enriched fish (e.g., tuna) or microalgae.

This invention was conducted with the support of a Collective Research Support Program funded by the Ministry of Science and ICT, entitled "Microbial engineering-based biosynthesis of human oxylipins based on metabolite profiling and elucidation of inflammation/obesity regulation mechanisms" (Project Identification Number: 1711154866; Performing Institution: Konkuk University).

### BACKGROUND ART

Dihydroxylated fatty acids (DiHFAs) having 20 to 22 carbon atoms are lipid regulators containing two hydroxyl groups, which are mainly present in animal lipids, including those of the human body. In vivo, it is biosynthesized from animal-derived polyunsaturated fatty acids (PUFAs) through combined reactions of aspirin-treated cyclooxygenase (COX), cytochrome P450 (CYP), and lipoxygenase (LOX). DiHFAs generated from eicosapentaenoic acid (EPA) and docosahexaenoic acid (DHA) belong to lipid regulators and are classified as specialized pro-resolving mediators (SPMs), which are a type of human signaling molecules that promote the resolution of inflammation. SPMs are endogenous regulators of infection and inflammation and play important roles in various physiological activities, such as microbial clearance, pain relief, tissue regeneration, anti-inflammatory responses, and homeostasis, in animals including humans. Inflammation-promoting mediators include leukotrienes (LTs) and prostaglandins (PGs), whereasinflammation-resolving mediators include protectin (PDs), resolvins (Rvs), maresins (MaRs), and lipoxins (LXs). Among these, resolvin represents an endogenous lipid mediator class that is generated through combined reactions and double-oxygenation reactions catalyzed by lipoxygenase. Rvs ares derived from omega fatty acids having 20 or 22 carbon atoms, such as eicosapentaenoic acid, docosapentaenoic acid, and docosahexaenoic acid.

Resolvin (Rvs) belongs to inflammation-resolving mediators that are mainly produced when lipoxygenases(LOXs) act on animal-derived unsaturated fatty acids, namely eicosapentaenoic acid and docosahexaenoic acid, thereby being converted into resolvin E and resolvin D, respectively. Rvs are known to be generated in trace amounts by macrophages during the resolution of inflammation and infection, in which they promote phagocytosis to remove viruses, bacteria, and inflammatory mediators, and facilitate the recovery of damaged tissues, thereby contributing to the resolution of inflammation and infection. Among these, resolvin D5 (RvD5) has been reported to exhibit diverse biological activities, including the ability to resolve inflammation even at trace levels in the human body, to reduce pain, and to decrease the required dosage of antibiotics in the treatment of infections, while also providing effects such as skin regeneration. In addition, resolvin D5 has been shown to reduce inflammation induced by intracellular oxidative stress, thereby exhibiting anti-inflammatory activity, and to suppress cell death through phagocytic activity, thereby contributing to the prevention of degeneration. Accordingly, if resolvin D5, which is an extremely high-cost specialized pro-resolving mediator (approximately 17,000,000 KRW per mg), can be mass-produced from a low-cost docosahexaenoic acid-enriched oil (80% (w/w), approximately 200,000 KRW per kg) through production optimization, industrialization thereof would be facilitated. Furthermore, resolvin D5 is suggested as a next-generation therapeutic material capable of addressing inflammation and infection, and is expected to be practically applicable in functional and efficacy studies in academic fields such as medicine and biotechnology.

Lipoxygenase (LOX) catalyzes stereospecific and regioselective peroxidation through a dioxygenating reaction using polyunsaturated fatty acids having one or more cis,cis-1,4-pentadiene moieties as substrates. Based on stereospecificity, lipoxygenase is classified according to whether the resulting chirality or handedness exhibits an R-form or an S-form, and is referred to as R-lipoxygenases and S-lipoxygenases, respectively. Regioselectivity varies depending on the carbon chain length of the polyunsaturated fatty acid used as a substrate and the position of the pentadiene structure. Among these, lipoxygenase generates hydroxyl groups at the 5-, 8-, 9-, 11-, 12-, and 15-carbon positions of arachidonic acid, which is an animal-derived polyunsaturated fatty acid, and generates hydroxyl groups at the 7-, 10-, 11-, 13-, 14-, and 17-carbon positions of C22 polyunsaturated fatty acids.

In the case of double lipoxygenases, hydroperoxides are generated at two carbon positions, and it is generally known that, through catalytic reactions, hydroxyl groups are formed while cis-cis structures are converted into cis-trans structures. Double lipoxygenases that have been identified to date include a mouse double arachidonate 8-lipoxygenase *(Mus musculus* double dioxygenating ARA 8-LOX), a *Sphingpyxis macrogoltabida* double arachidonate 9-lipoxygenase (*Sphingpyxis macrogoltabida* ARA 9-LOX), an *Endozoicomonas numazuensis* double arachidonate 12-lipoxygenase *(Endozoicomonas numazuensis* ARA 12-LOX), and a double arachidonate 15-lipoxygenase derived from *Archangium violaceum* (*Archangium violaceum* ARA 15-LOX). Dihydroxylated fatty acids formed by double 8-lipoxygenases in mammals are biosynthesized in vivo through intercellular responses to tissue injury and infection, and resolvin D5 is produced only in trace amounts, at levels of 250 pg/mL or less. It has been reported that the production of dihydroxylated fatty acids using whole cells expressing three types of bacterial-derived lipoxygenases is carried out through a one-step biotransformation. Among these enzymes, a double arachidonate 15-lipoxygenase derived from *Archangium violaceum* exhibits the highest activity and is capable of producing specialized pro-resolving mediators; however, production at the g/L level has not been achieved to date, and the production of specialized pro-resolving mediators in a bioreactor through whole-cell reactions has not been reported. In the case of the double arachidonate 15-lipoxygenase derived from *Archangium violaceum,* S-hydroxyl groups are selectively formed at the 5- and 15-carbon positions in C20 unsaturated fatty acids such as arachidonic acid. In addition, in C22 unsaturated fatty acids, S-hydroxyl groups are formed at the 7- and 17-carbon positions.

To date, reported methods for synthesizing resolvin D5 include a 25-step chemical synthesis process that affords a yield of approximately 20%, and such methods have limitations in that benzene homologs and heavy metals are used and released during the synthesis process, thereby causing environmental pollution. In addition, it has been reported that resolvin D5 is biosynthesized at a level of approximately 0.1 g/L by a double arachidonate 15-lipoxygenase derived from *Archangium violaceum* using whole-cell systems. However, in whole-cell biotransformation processes, oxidative reactions are inhibited by substrates and produced intermediates at or above certain concentrations, and it is known that reaction inhibition caused by hydrophobic fatty acids can be overcome by providing sufficient oxygen supply and using solvents during catalytic reactions.

In the present invention, a substrate is secured by effectively hydrolyzing a docosahexaenoic acid (DHA)-enriched oil into polyunsaturated fatty acids including DHA using a commercial lipase and an adsorption resin. Using the hydrolysate as a substrate, reaction conditions for conversion into resolvin D5, an endogenous substance in the human body, are optimized through whole-cell reactions employing a double arachidonate 15-lipoxygenase derived from the bacterium *Archangium violaceum,* in both flask-scale and bioreactor systems, thereby achieving biosynthesis of resolvin D5 at the g/L level with a high conversion yield of 70% or more and a purification yield of 97% or more. In addition, maximum conversion was confirmed by using high-purity DHA reagents of 95% and 99% as substrates. To date, there have been no reports of optimization and production of resolvin D5 in flasks and bioreactors using, as substrates, polyunsaturated fatty acids obtained by hydrolysis followed by solvent extraction from low-cost DHA-enriched oil, or high-purity DHA reagents, for the production of resolvin D5.

### DISCLOSURE

### TECHNICAL PROBLEM

The present invention has been devised in view of the above-described needs, and an object of the present invention is to provide a composition for producing dihydroxylated fatty acids having 20 to 22 carbon atoms, the composition comprising, at a high purity, polyunsaturated fatty acids derived from a DHA-containing oil obtained from fish or microalgae.

The present invention provides a composition for producing dihydroxylated fatty acids having 20 to 22 carbon atoms, the composition comprising a lipase for hydrolyzing a low-cost DHA-containing oil obtained from fish or microalgae into polyunsaturated fatty acids including docosahexaenoic acid at a high purity, and an adsorption resin.

The present invention also aims to provide a method for purifying docosahexaenoic acid at a high purity (approximately 70 to 99%) by subjecting a hydrolysate produced by the above-described hydrolysis, which comprises polyunsaturated fatty acids including docosahexaenoic acid, to an additional solvent extraction method. In the solvent extraction of the present invention, extraction is preferably performed using a solvent in which hexane and acetonitrile are mixed at a volume ratio of 3 to 5:1 (more preferably 4:1), and any extraction method belonging to the relevant technical field may be used without limitation.

The present invention also provides a method for producing resolvin D5 by biotransformation using, as a substrate, a composition comprising high-purity docosahexaenoic acid. The present invention further aims to provide a composition and a method for producing resolvin D5, an endogenous substance in the human body, by using the substrate and a double 15-lipoxygenase derived from *Archangium violaceum.*

Specifically, the present invention is directed to providing a method for producing resolvin D5, an endogenous substance in the human body, by treating a microorganism transformed with a gene encoding a double 15-lipoxygenase derived from *Archangium violaceum* with high-purity docosahexaenoic acid reagents having a purity of 95% or 99% as a substrate.

Accordingly, the present invention provides a composition comprising high-purity polyunsaturated fatty acids, including docosahexaenoic acid, which is obtained by hydrolyzing and purifying an oil derived from fish or microalgae using a commercial lipase and an adsorption resin. The present invention further provides a composition for producing resolvin D5 at a high yield through a biotransformation process using, as a substrate, the composition comprising the high-purity polyunsaturated fatty acids, as well as a method for producing resolvin D5 using the substrate. In addition, the present invention provides resolvin D5 produced by the method.

Another object of the present invention is to provide a composition for producing dihydroxylated fatty acids or resolvin D5, the composition comprising the enzyme as an active ingredient.

Another object of the present invention is to provide a method for obtaining polyunsaturated fatty acids serving as a substrate from an oil obtained from fish or microalgae, and for producing dihydroxylated fatty acids or resolvin D5 using the same.

However, the technical problems to be solved by the present invention are not limited to those mentioned above, and other technical problems not mentioned herein will be clearly understood by those skilled in the art from the following description.

### TECHNICAL SOLUTION

The terminology used herein is for the purpose of description only and is not intended to be limiting. Unless the context clearly indicates otherwise, singular expressions include plural expressions. As used herein, the terms "comprise," "include," or "have" are intended to specify the presence of stated features, numbers, steps, operations, components, parts, or combinations thereof, but do not preclude the presence or addition of one or more other features, numbers, steps, operations, components, parts, or combinations thereof. Unless otherwise defined, all terms used herein, including technical and scientific terms, have the same meanings as those commonly understood by a person having ordinary skill in the art to which the embodiments belong. Terms defined in generally used dictionaries are to be interpreted as having meanings consistent with their meanings in the context of the relevant art and the present disclosure, and are not to be interpreted in an idealized or overly formal sense unless expressly so defined herein.

It is known that resolvin D5 can be used as a research material in various academic fields such as medicine, biology, and biotechnology. However, a method has not yet been developed for producing resolvin D5 at a high yield of 70% or more and at the g/L level by a biological process in flasks and bioreactors using, as a substrate, high-purity docosahexaenoic acid or polyunsaturated fatty acids obtained by hydrolyzing a low-cost docosahexaenoic acid-enriched oil. In addition, a method for purifying docosahexaenoic acid at a high purity of 97% or more from an oil obtained from fish or microalgae has not yet been developed.

In order to achieve the above-described technical problems, the inventors of the present invention hydrolyzed a low-cost oil obtainable from fish or microalgae (a docosahexaenoic acid-enriched oil) into polyunsaturated fatty acids containing docosahexaenoic acid at a content of 70% or more (about 70% to 80% or 70% to 99%) by using a commercial lipase and an adsorption resin, and then secured high-purity docosahexaenoic acid (about 90%) by a solvent extraction method. Using, as a substrate, a composition comprising the obtained high-purity docosahexaenoic acid, continuous studies were conducted to more effectively produce resolvin D5, which is a dihydroxylated fatty acid, through an optimized biotransformation process. Specifically, the inventors of the present invention isolated docosahexaenoic acid, which serves as a substrate in a biotransformation process, from a docosahexaenoic acid-enriched oil and purified the same to a purity of 90%. Using, as a substrate, a composition comprising the purified high-purity docosahexaenoic acid, resolvin D5, which is a final product, was produced by an environmentally friendly biotransformation method through whole-cell reactions in flasks and bioreactors employing a double 15-lipoxygenase derived from *Archangium violaceum,* wherein the resolvin D5 is 7S,17S-dihydroxy-4Z,8E,10Z,13Z,15E,19Z-docosahexaenoic acid. Thereafter, in an optimized biotransformation process, the inventors confirmed that by treating the reaction system with a solvent and a polymer, the solubility of the substrate could be efficiently increased, thereby enabling production with high productivity and high yield through sufficient oxygen supply in a bioreactor, and thus completed the present invention.

Accordingly, the present invention provides a composition comprising, as a substrate, polyunsaturated fatty acids at a high purity derived from a DHA-containing oil obtained from sources such as fish or microalgae, for use in producing dihydroxylated fatty acids having 20 to 22 carbon atoms.

In the composition, the polyunsaturated fatty acid derived from the DHA-containing oil may comprise docosahexaenoic acid (DHA).

The polyunsaturated fatty acid obtained after hydrolyzing the composition may comprise docosahexaenoic acid at a high purity.

As used herein, the term "oil" includes any DHA-containing oil derived from microalgae, fungi, fish, and the like.

As used herein, the term "dihydroxylated fatty acid" refers to a fatty acid, preferably an unsaturated fatty acid having 20 to 22 carbon atoms, in which two hydroxyl groups are substituted.

As used herein, dihydroxylated fatty acids having 20 to 22 carbon atoms include resolvin D5.

As used herein, resolvin D5 is a compound represented by Chemical Formula 1, and is named 7S,17S-dihydroxy-4Z,8E,10Z,13Z,15E,19Z-docosahexaenoic acid, and is also referred to as 7S,17S-dihydroxydocosahexaenoic acid (DiHDHA).

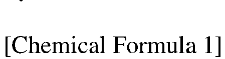

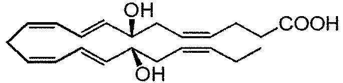

The present invention also provides a composition comprising docosahexaenoic acid obtained by hydrolyzing a DHA-containing oil derived from fish or microalgae, as well as a method for decomposing and purifying an oil derived from fish or microalgae into polyunsaturated fatty acids.

In addition, the present invention provides a method for producing dihydroxylated fatty acids having 20 to 22 carbon atoms (resolvin D5), the method comprising: (1) hydrolyzing a low-cost DHA-containing oil obtained from fish or microalgae using a commercial lipase and an adsorption resin; (2) purifying the obtained hydrolysate by a solvent extraction method to obtain a composition comprising docosahexaenoic acid at a high purity (about 70% to 99%); and (3) producing resolvin D5 through a whole-cell reaction by reacting a transformed microorganism comprising a gene encoding a double 15-lipoxygenase using the purified composition as a substrate. The present invention further provides a method for purifying dihydroxylated fatty acids having 20 to 22 carbon atoms (resolvin D5) to a high purity. In addition, the present invention provides optimized conditions for producing resolvin D5 through whole-cell reactions in a bioreactor.

After step (3), the present invention further comprises a purification step (4) for producing resolvin D5 at a high yield. According to the purification method, resolvin D5 can be obtained at a high purity of 90% or more (preferably 99% or more). The purification step (4) may be performed simultaneously or sequentially by ultrafiltration, column filtration, affinity chromatography, and reversed-phase chromatography. Preferably, the purification step is performed in the order of ultrafiltration, reversed-phase chromatography, (preparative column filtration), and affinity chromatography.

In the affinity chromatography, an adsorption resin selected from HP20, HP2MG, SP207, SP825, SP850, or XAD761 may be used, and in the reversed-phase chromatography, a C1 (TMS) resin, a C4 (butyl) resin, a C8 (octyl) resin, or a C18 (octadecyl) resin may be used; however, the present invention is not limited thereto.

Specifically, the present invention provides a purification method for producing a hydrolysate (polyunsaturated fatty acids comprising docosahexaenoic acid) by hydrolyzing the low-cost docosahexaenoic acid-enriched oil using a commercial lipase and an adsorption resin, and for obtaining docosahexaenoic acid having a purity of 90%, as well as a composition comprising the same.

The present invention provides a composition for producing dihydroxylated fatty acids having 20 to 22 carbon atoms (preferably, resolvin D5) by reacting the purified hydrolysate (polyunsaturated fatty acids comprising docosahexaenoic acid) as a substrate with a double 15-lipoxygenase.

The present invention provides a composition comprising, as an active ingredient, docosahexaenoic acid having a high purity of 95% or 99%, wherein the docosahexaenoic acid is reacted as a substrate with a double 15-lipoxygenase, for use in producing resolvin D5, which is a dihydroxylated fatty acid.

In addition, the present invention provides a composition for producing resolvin D5, wherein polyunsaturated fatty acids comprising docosahexaenoic acid are obtained as a hydrolysate by hydrolyzing an oil obtained from fish or microalgae using a commercial lipase and an adsorption resin, and resolvin D5 is produced by reacting the obtained docosahexaenoic acid having a purity of 90% and high-purity docosahexaenoic acid having a purity of 95% as substrates with a double 15-lipoxygenase, followed by purifying the produced resolvin D5 to a high purity using ultrafiltration, octadecyl-silica (ODS) resin, and preparative liquid chromatography (prep-LC) containing a C18 resin.

In one embodiment of the present invention, the adsorption resin included in the composition may be an adsorption resin that binds to fatty acids during the reaction to reduce substrate inhibition, and may be selected from HP20, HP2MG, SP207, SP825, SP850, and XAD761, preferably SP207.

In one embodiment of the present invention, the production method preferably uses a DHA-enriched oil, which serves as a substrate, at a concentration of 10 g/L.

In one embodiment of the present invention, in the production method, the concentration of the adsorption resin SP207 is preferably 10 g/L; however, any adsorption resin may be used without limitation, provided that it is capable of binding to fatty acids. Specifically, SP207 may be used, but the present invention is not limited thereto.

In one embodiment of the present invention, in the production method, the concentration of a commercial lipase, specifically a lipase derived from *Thermomyces lanuginosus,* is preferably 1000 U/mL. However, any commercial lipase may be used, provided that it performs the function of hydrolyzing an oil as a substrate into polyunsaturated fatty acids. Specifically, a lipase derived from *Thermomyces lanuginosus* may be used, but the present invention is not limited thereto.

In addition, the production method is preferably performed at a pH in the range of 6 to 9 (for example, pH 8 to 8.5, preferably pH 8.5) and at a temperature in the range of 10 to 40 °C (preferably 20 °C or 30 °C). In order to maintain such pH conditions, an EPPS buffer solution may be used as a reaction solvent.

In one embodiment, in the solvent extraction and purification method, a volume ratio of hexane to acetonitrile of 4:1 (v/v) is preferred.

By maintaining such conditions, productivity and purification yield can be improved in producing a composition comprising polyunsaturated fatty acids (docosahexaenoic acid) at a high purity from an oil obtained from fish or microalgae.

The present invention provides a method for producing resolvin D5, which is a dihydroxylated fatty acid, the method comprising treating a recombinant strain expressing a double 15-lipoxygenase with a composition obtained by hydrolyzing and purifying an oil obtained from fish or microalgae into polyunsaturated fatty acids comprising docosahexaenoic acid through whole-cell reactions in flasks and bioreactors.

The present invention provides a method for producing resolvin D5, which is a dihydroxylated fatty acid, the method comprising treating a recombinant strain expressing a double 15-lipoxygenase with high-purity docosahexaenoic acid reagents having a purity of 95% or 99% as a substrate through whole-cell reactions in flasks and bioreactors.

In the present invention, the double 15-lipoxygenase derived from *Archangium violaceum* comprises an amino acid sequence of SEQ ID NO: 1 or a sequence having at least 80% sequence identity thereto, and SEQ ID NO: 2 represents a gene sequence encoding the same.

In one embodiment, in the production method, the substrate may further comprise at least one selected from the group consisting of a docosahexaenoic acid-enriched oil, high-purity docosahexaenoic acid reagents having a purity of 95% or 99%, or a hydrolysate obtained by hydrolyzing and purifying a docosahexaenoic acid-enriched oil, wherein the hydrolysate comprises polyunsaturated fatty acids including, in addition to docosahexaenoic acid as a main component, eicosapentaenoic acid, docosapentaenoic acid, and arachidonic acid.

In one embodiment, the composition may further comprise a solvent and a polymer that efficiently increase the solubility of fatty acids and the substrate during the reaction, and preferably dimethyl sulfoxide (DMSO) and polyvinylpyrrolidone (PVP) may be used.

The whole-cell reaction is preferably performed at a temperature of about 20 °C. In order to maintain such pH conditions, an EPPS buffer solution may be used as a reaction solvent.

In addition, in the production method, *Escherichia coli* transformed with a recombinant expression vector comprising a gene encoding a double 15-lipoxygenase may be cultured to induce expression of the recombinant enzyme gene, and the resulting strain containing the expressed protein may be harvested and used. It is preferable to obtain and use the strain in this manner. In addition, the duration of the whole-cell reaction may be appropriately adjusted according to conventional methods.

In another embodiment of the present invention, the production method preferably involves treating cells comprising the double 15-lipoxygenase at a concentration in the range of 0.5 g/L to 6.0 g/L; however, the present invention is not limited thereto.

In the production method, it is preferable to treat docosahexaenoic acid at a concentration in the range of 1 mM to 9 mM, wherein the docosahexaenoic acid is selected from the above-described high-purity docosahexaenoic acid reagents having a purity of 95% or 99%, or a hydrolysate obtained by hydrolyzing and purifying a docosahexaenoic acid-enriched oil and having a purity of 90%; however, the present invention is not limited thereto.

In the production method, the concentration of a solvent, dimethyl sulfoxide (DMSO), is preferably in the range of 0.1% to 10%; however, any solvent may be used without limitation, and the present invention is not limited thereto.

In the production method, the concentration of a polymer, polyvinylpyrrolidone (PVP), is preferably in the range of 0.1% to 10%; however, any polymer may be used without limitation, and the present invention is not limited thereto.

In the production method, the concentration of isopropyl-thiogalactopyranoside (IPTG) is preferably 0.1 mM; however, the present invention is not limited thereto.

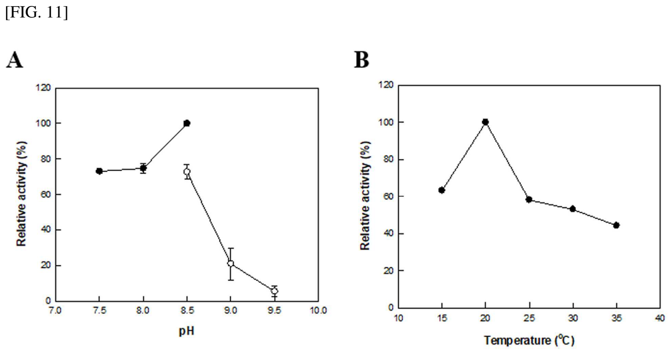

In addition, the production method is preferably performed at a pH in the range of 6 to 9 (for example, pH 8 to 8.5, preferably pH 8.5). In order to perform the reaction, an EPPS buffer solution may be used as a reaction solvent. Referring to FIG. 11A, it is appropriate to perform the reaction at a pH in the range of 6 to 9 (for example, pH 8 to 8.5, preferably pH 8.5) depending on the reaction activity conditions of HEPPS and CHES.

The whole-cell reaction is preferably performed at a temperature in the range of 10 to 40 °C (about 20 °C). Referring to FIG. 11B, when a reaction was performed for 30 minutes at various temperatures ranging from 15 °C to 35 °C in a 50 mM HEPPS buffer (pH 8.5) containing 0.5 g L⁻¹ cells and 3 mM arachidonic acid (ARA), the reaction activity was higher when the reaction was performed at a temperature in the range of 10 to 40 °C (preferably 15 to 35 °C, more preferably 15 to 25 °C).

The purification method preferably involves treating resolvin D5, which is produced from a substrate selected from high-purity docosahexaenoic acid reagents having a purity of 95% or a hydrolysate having a purity of 90% obtained by hydrolyzing and purifying a docosahexaenoic acid-enriched oil, at a concentration of about 10 g/L; however, the present invention is not limited thereto.

In one embodiment, in the purification method, purification is preferably performed using ultrafiltration, C18 octadecyl resin, a preparative column, and an adsorption resin SP825; however, the present invention is not limited thereto.

In one embodiment, in the purification method, the concentration of the adsorption resin SP825 is preferably 10 g/L; however, any adsorption resin may be used, provided that it performs the function of binding to resolvin D5. Specifically, SP825 may be used, but the present invention is not limited thereto.

As described above, according to the present invention, docosahexaenoic acid having a purity of 90% was secured through a hydrolysate obtained by hydrolyzing and purifying an oil obtained from fish or microalgae into polyunsaturated fatty acids comprising docosahexaenoic acid using a commercial lipase and an adsorption resin. Using, as a substrate, the obtained docosahexaenoic acid having a purity of 90%, as well as high-purity docosahexaenoic acid reagents having a purity of 95% or 99%, resolvin D5 (7S,17S-dihydroxy-4Z,8E,10Z,13Z,15E,19Z-docosahexaenoic acid), which is a final product, was produced by an environmentally friendly biotransformation method through whole-cell reactions of a microorganism transformed with a double 15-lipoxygenase derived from *Archangium violaceum.* In the biotransformation process, treatment with a solvent and a polymer efficiently increased the solubility of the substrate, thereby enabling production with high productivity and high yield through sufficient oxygen supply in a bioreactor. The produced resolvin D5 was subsequently purified to a high purity using ODS and C18 resins. As a result, by optimizing the reaction for producing resolvin D5, which is an endogenous substance in the human body, from a low-cost oil obtained from fish or microalgae, resolvin D5 was produced at a high yield of 70% or more and a purification yield of 90% or more at the g/L level, and could be purified to a purity of 97% or more.

### ADVANTAGEOUS EFFECTS

Resolvin D5, which is an endogenous substance in the human body and is produced according to the present invention, functions as a signaling molecule and may be involved in various physiological activities in animals, including humans. When the production method of the present invention is employed, resolvin D5 can be produced with higher productivity and yield than conventional methods and can be purified to a high purity, and thus may be usefully applied in various industrial fields, including pharmaceuticals, foods, and cosmetics.

### DESCRIPTION OF DRAWINGS

FIG. 1 illustrates the results of resolvin D5 production amounts depending on the concentrations of dimethyl sulfoxide (DMSO) as a solvent and polyvinylpyrrolidone (PVP) as a polymer, for maximum production of resolvin D5 generated by whole-cell reactions of a microorganism transformed with a double 15-lipoxygenase derived from *Archangium violaceum,* using high-purity docosahexaenoic acid reagents having a purity of 99% as a substrate.
FIG. 2 illustrates the results of resolvin D5 production amounts depending on the concentrations of whole cells and substrate in whole-cell reactions for maximum production of resolvin D5, using high-purity docosahexaenoic acid reagents having a purity of 99% as a substrate.
FIG. 3 illustrates the results of resolvin D5 production amounts according to the types of solvents and polymers for maximum production of resolvin D5 when biotransformation was performed using, as a substrate, polyunsaturated fatty acids comprising docosahexaenoic acid having a purity of 90%, which were obtained by hydrolyzing an oil derived from fish or microalgae followed by solvent extraction. The solvents used include isopropyl alcohol (IPA), methanol (MeOH), ethanol (EtOH), ethyl acetate (EA), and dimethyl sulfoxide (DMSO), and the polymers used include polyvinylpyrrolidone (PVP), polyvinyl alcohol (PVA), and polyethylene glycol (PEG).
FIG. 4 illustrates the results of resolvin D5 production amounts depending on the concentrations of dimethyl sulfoxide (DMSO) as a solvent and polyvinylpyrrolidone (PVP) as a polymer for maximum production of resolvin D5 generated by whole-cell reactions of a microorganism transformed with a double 15-lipoxygenase derived from *Archangium violaceum,* using, as a substrate, polyunsaturated fatty acids comprising docosahexaenoic acid having a purity of 90%, which were obtained by hydrolyzing an oil derived from fish or microalgae followed by solvent extraction.
FIG. 5 illustrates the results of resolvin D5 production amounts depending on the concentrations of whole cells and substrate for maximum production of resolvin D5 generated using docosahexaenoic acid having a purity of 90% in a hydrolysate as a substrate.
FIG. 6 illustrates the results of resolvin D5 production amounts depending on the concentrations of whole cells and substrate for maximum production of resolvin D5 generated by whole-cell reactions in a bioreactor for large-scale production, using high-purity docosahexaenoic acid reagents having a purity of 95% as a substrate.
FIG. 7 illustrates the results of time-dependent production amounts of resolvin D5 generated by recombinant *Escherichia coli* expressing a double 15-lipoxygenase derived from *Archangium violaceum* in a bioreactor for large-scale production, using high-purity docosahexaenoic acid reagents having a purity of 95% as a substrate.
FIG. 8 illustrates the results of resolvin D5 production amounts depending on the concentrations of whole cells and substrate for maximum production of resolvin D5 generated by whole-cell reactions in a bioreactor for large-scale production, using, as a substrate, docosahexaenoic acid having a purity of 90% contained in a hydrolysate of an oil obtained from fish or microalgae.
FIG. 9 illustrates the results of time-dependent production amounts of resolvin D5 generated by recombinant *Escherichia coli* expressing a double 15-lipoxygenase derived from *Archangium violaceum* in a bioreactor, using, as a substrate, docosahexaenoic acid having a purity of 90% contained in a hydrolysate of an oil obtained from fish or microalgae.

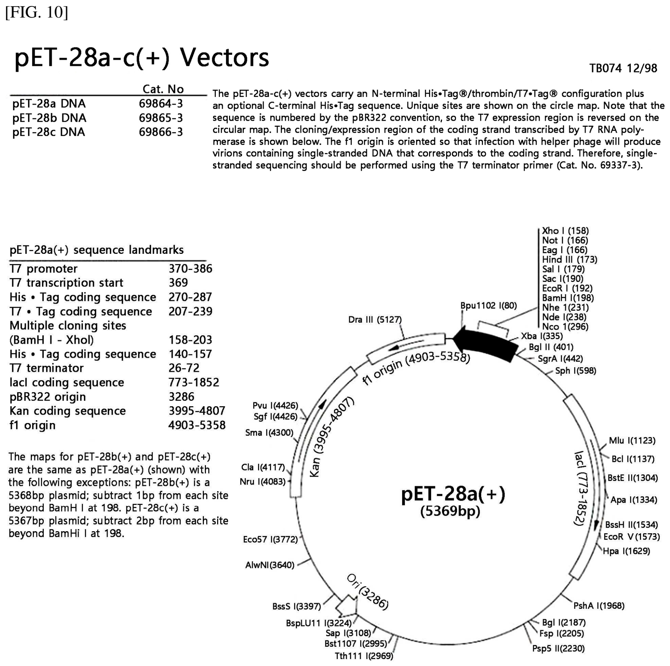

FIG. 10 illustrates a recombinant expression vector constructed to express a double 15-lipoxygenase derived from *Archangium violaceum* according to the present invention.
FIG. 11 illustrates the results of evaluating the effects of pH, temperature, and cysteine concentration on the production of 5S,15S-DiHETE. FIG. 11A illustrates the effect of pH on the production of 5S,15S-DiHETE (5S,15S-dihydroxyeicosatetraenoic acid). The reaction was performed for 30 minutes at 20 °C in 50 mM 3-[4-(2-hydroxyethyl)piperazin-1-yl]propane-1-sulfonic acid (HEPPS) buffer and 50 mM N-cyclohexyl-2-aminoethanesulfonic acid (CHES) buffer, while varying the pH from 7.5 to 9.5, each buffer containing 0.5 g L⁻¹ cells and 3 mM arachidonic acid (ARA). In FIG. 11A, the symbols ● and ○ represent 50 mM HEPPS and 50 mM CHES, respectively. FIG. 11B illustrates the effect of temperature on the production of 5S,15S-DiHETE. In this example, the reaction was performed for 30 minutes at various temperatures ranging from 15 °C to 35 °C in a 50 mM HEPPS buffer (pH 8.5) containing 0.5 g L⁻¹ cells and 3 mM arachidonic acid (ARA).

### BEST MODES OF THE INVENTION

Hereinafter, preferred embodiments are presented to facilitate understanding of the present invention. However, the following embodiments are provided merely for ease of understanding of the present invention, and the scope of the present invention is not limited by these embodiments.

### [Example 1]

### Preparation of Double 15-Lipoxygenase

The transformed microorganisms were cultured in 1 L of Luria-Bertani (LB; Difco, Sparks, MD, USA) medium containing 20 µg/mL kanamycin in shaking Erlenmeyer flasks under aeration conditions at 200 rpm and 37 °C. When the optical density of the whole cells reached 0.6 to 0.8 at 600 nm, isopropyl-β-D-thiogalactopyranoside (IPTG) was added at a final concentration of 0.1 mM to induce protein expression of the enzyme, and the culture was further incubated at 16 °C for 16 hours with shaking at 150 rpm. The double 15-lipoxygenase overexpressed and produced as described above was obtained by centrifuging the culture broth of the transformed strain at 6,000 × g for 30 minutes at 4 °C, washing the collected cells twice with 0.85% sodium chloride (NaCl), and then using the washed cells as recombinant cells for producing resolvin D5.

### [Example 2]

### Production of Resolvin D5 from 99% Pure Docosahexaenoic Acid Reagent Using Double 15-Lipoxygenase

In order to mass-produce resolvin using the above-described double 15-lipoxygenase, a reaction was performed using docosahexaenoic acid as a substrate. To determine the optimal concentrations of dimethyl sulfoxide (DMSO) as a solvent and polyvinylpyrrolidone (PVP) as a polymer for maximum production of resolvin D5 generated by whole-cell reactions of a microorganism transformed with a double 15-lipoxygenase derived from *Archangium violaceum, a* whole-cell reaction was performed for 60 minutes at pH 8.5 and 20 °C, including 3 mM docosahexaenoic acid and 1 g/L whole cells.

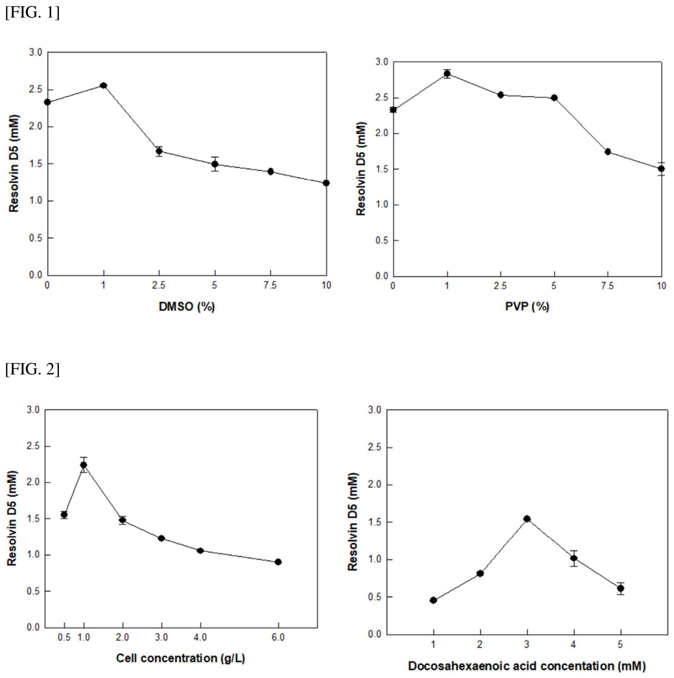

As a result, it was confirmed in FIG. 1 that the optimal concentrations of dimethyl sulfoxide as the solvent and polyvinylpyrrolidone as the polymer were each 1%. In addition, under the optimal concentrations of the solvent and polymer, maximum production was confirmed through optimization of the concentration of recombinant *Escherichia coli* expressing the double 15-lipoxygenase and the concentration of a docosahexaenoic acid reagent having a purity of 99% as a substrate. As shown in FIG. 2, the optimal concentrations of the recombinant *Escherichia coli* expressing the double 15-lipoxygenase and the docosahexaenoic acid reagent having a purity of 99% as the substrate were 1 g/L and 3 mM, respectively. It was confirmed that approximately 2.24 mM (0.81 g/L) of resolvin D5 was produced in a flask by a whole-cell reaction using cells expressing the double 15-lipoxygenase (FIG. 2).

### [Example 3]

### Hydrolysis and Purification of Polyunsaturated Fatty Acids from a Docosahexaenoic Acid-Enriched Fish Oil

An oil obtained from low-cost fish or microalgae was used at a concentration of 10 g/L and hydrolyzed into polyunsaturated fatty acids using a commercial lipase at 1000 U/mL and an adsorption resin SP207 at 10 g/L, while stirring at 250 rpm at 30 °C for 24 hours, thereby producing polyunsaturated fatty acids containing docosahexaenoic acid at a purity of 70%. In order to increase the purity, the hydrolysate was mixed with hexane and acetonitrile at a volume ratio of 4:1 (v/v), stirred for 4 minutes, stored at -20 °C for 2 hours, and then the lower acetonitrile layer was collected to secure a polyunsaturated fatty acid substrate containing docosahexaenoic acid at a purity of 90% (Table 1).

Table 1 below shows the results of hydrolysis and purification by solvent extraction into polyunsaturated fatty acids comprising docosahexaenoic acid at a purity of 90%, using a commercial lipase and an adsorption resin from an oil obtained from fish or microalgae.

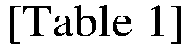

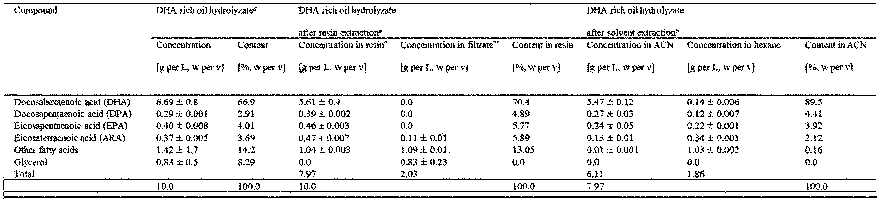

### [Example 4]

### Synthesis of Resolvin D5 Using, as a Substrate, Polyunsaturated Fatty Acids Comprising Docosahexaenoic Acid Having a Purity of 90%, Obtained by Hydrolysis and Subsequent Solvent Extraction from a Docosahexaenoic Acid-Enriched Oil

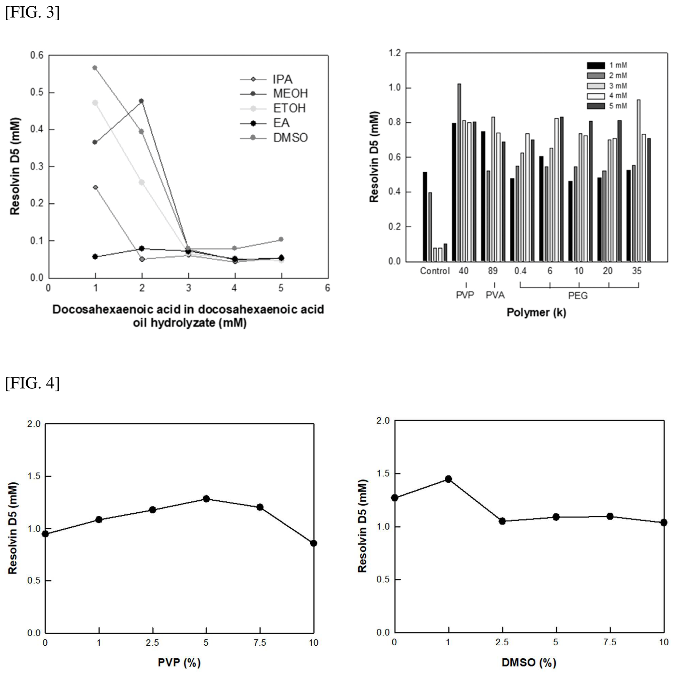

Using the above-described double 15-lipoxygenase, optimal production conditions for resolvin D5 were investigated using, as a substrate, polyunsaturated fatty acids comprising docosahexaenoic acid having a purity of 90%, which were obtained by hydrolysis and subsequent solvent extraction from a docosahexaenoic acid-enriched oil. In order to identify optimal types of solvent and polymer suitable for production, a whole-cell reaction was performed for 60 minutes at pH 8.5 and 20 °C using 2 mM docosahexaenoic acid having a purity of 90% as a substrate and 1.0 g/L whole cells transformed with a recombinant double 15-lipoxygenase. As a result, it was confirmed in FIG. 3 that dimethyl sulfoxide as a solvent and polyvinylpyrrolidone as a polymer were optimal additive types for the production of resolvin D5. Subsequently, a whole-cell reaction was performed for 60 minutes at pH 8.5 and 20 °C using 2.0 mM docosahexaenoic acid having a purity of 90% obtained by hydrolysis and solvent extraction as a substrate and 1 g/L whole cells transformed with the recombinant double 15-lipoxygenase. It was confirmed that the optimal concentrations of the additives were 1.0% dimethyl sulfoxide and 5% polyvinylpyrrolidone, respectively (FIG. 4).

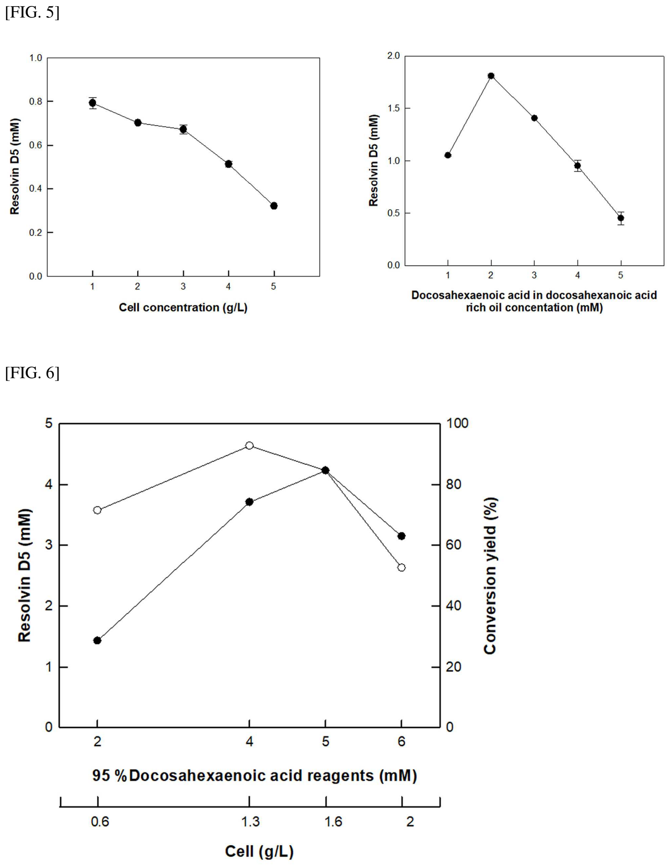

In addition, under the optimal concentrations of the solvent and polymer, maximum production was confirmed by optimizing the concentration of recombinant *Escherichia coli* expressing the double 15-lipoxygenase and the concentration of the substrate, which was polyunsaturated fatty acids comprising docosahexaenoic acid having a purity of 90% obtained by hydrolysis and solvent extraction from an oil derived from fish or microalgae. As shown in FIG. 5, the optimal concentrations of the recombinant *Escherichia coli* expressing the double 15-lipoxygenase and the substrate, which was docosahexaenoic acid having a purity of 90% obtained by hydrolysis and subsequent solvent extraction, were 1 g/L and 2 mM, respectively. It was confirmed that approximately 1.8 mM (0.65 g/L) of resolvin D5 was produced in a flask by a whole-cell reaction using cells expressing the double 15-lipoxygenase (FIG. 5).

### [Example 5]

### Large-Scale Production of Resolvin D5 in a Bioreactor Using Whole-Cell Reactions of Double 15-Lipoxygenase with High-Purity Docosahexaenoic Acid Reagents Having a Purity of 95% and Polyunsaturated Fatty Acids Comprising Docosahexaenoic Acid Having a Purity of 90% Obtained by Hydrolysis and Solvent Extraction from an Oil Derived from Fish or Microalgae as Substrates

Whole-cell reactions of double 15-lipoxygenase were carried out in a 3 L bioreactor using 1 L of culture broth containing recombinant *Escherichia coli* expressing the double 15-lipoxygenase, with substrates including a high-purity docosahexaenoic acid reagent having a purity of 95% and polyunsaturated fatty acids comprising docosahexaenoic acid having a purity of 90%, which were obtained by hydrolysis and subsequent solvent extraction from a docosahexaenoic acid-enriched oil derived from fish or microalgae. Under optimal conditions of 1.0% dimethyl sulfoxide and 1.0% polyvinylpyrrolidone, a whole-cell reaction was performed using the high-purity docosahexaenoic acid reagent having a purity of 95% as a substrate at pH 8.5 and 20 °C for 6 hours with agitation at 1000 rpm and an aeration rate of 1.0 vvm. In addition, under conditions of 1.0% dimethyl sulfoxide and 5.0% polyvinylpyrrolidone, whole-cell reactions were performed using, as a substrate, docosahexaenoic acid having a purity of 90% obtained by hydrolysis and subsequent solvent extraction from an oil, and the extent of resolvin D5 production was evaluated through optimization of whole-cell concentration and substrate concentration in the 3 L bioreactor.

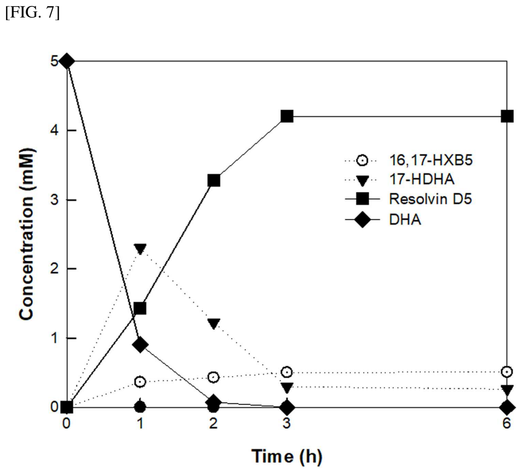

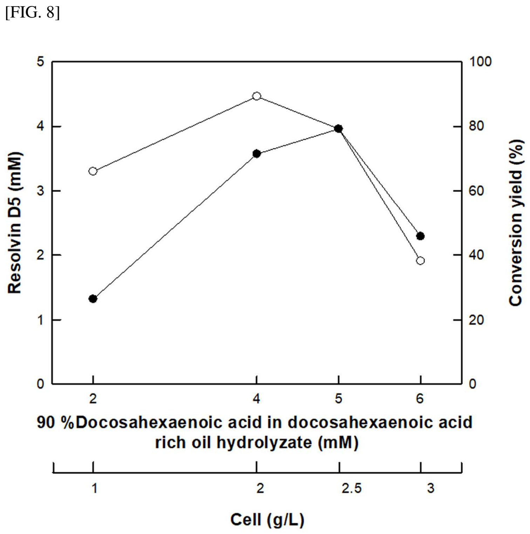

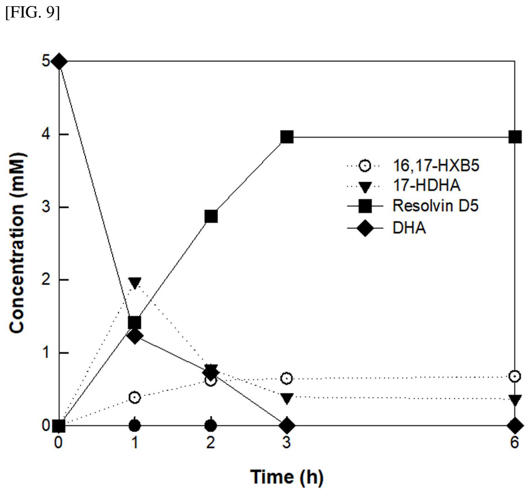

As a result, as shown in FIG. 6, when 1.6 g/L of whole cells was treated with 5.0 mM of the high-purity docosahexaenoic acid reagent having a purity of 95% as a substrate, the highest concentration of resolvin D5 was produced, and it was confirmed that up to approximately 4.21 mM (1.52 g/L) of resolvin D5 was generated by the whole-cell reaction using cells expressing the double 15-lipoxygenase (FIG. 7). In addition, as shown in FIG. 8, when 2.5 g/L of whole cells was treated with 5.0 mM of docosahexaenoic acid having a purity of 90% obtained from the hydrolysate as a substrate, the highest concentration of resolvin D5 was produced, and it was confirmed that up to approximately 3.97 mM (1.43 g/L) of resolvin D5 was generated by the whole-cell reaction using cells expressing the double 15-lipoxygenase (FIG. 9). These results demonstrate that the use of a 3 L bioreactor providing sufficient oxygen supply increased resolvin D5 production by approximately 197% and 217%, respectively, compared to production in flasks.

### [Example 6]

### Purification and Yield Evaluation of Resolvin D5 Produced at a Large Scale Using Ultrafiltration, C18 Octadecyl Resin, Preparative Column Chromatography, and an Adsorption Resin

In order to increase the purity of resolvin D5 contained in 1 L of a reaction mixture produced at a large scale using the bioreactor, purification was carried out through a total of four steps using ultrafiltration, a C18 octadecyl resin, preparative column chromatography, and an adsorption resin SP825 (Table 2). First, to remove impurities and high-molecular-weight proteins, ultrafiltration was performed using a 10 kDa filter by centrifugation at 6,000 × g for 30 minutes at 4 °C. The material obtained by ultrafiltration, as a first purified fraction, was loaded onto a C18 octadecyl resin that had been equilibrated with tertiary distilled water, washed with a 50% acetonitrile solvent, and thereafter resolvin D5 was purified and obtained using a 60% acetonitrile solvent. The resolvin D5 obtained after the second purification step using the C18 octadecyl resin was further purified using preparative column chromatography by applying a concentration gradient of 50% to 100% acetonitrile at a flow rate of 6 mL/min for 30 minutes at 30 °C, and the purified fraction was collected at absorbance of 202 nm. Finally, the previously purified resolvin D5 was mixed with an adsorption resin SP825 at a concentration of 10 g/L under agitation at 200 rpm at 40 °C for 3 hours. The adsorption resin was then recovered using a 45 µm filter, and resolvin D5 adsorbed onto the resin was eluted with ethyl acetate and recovered. As a result of the four-step purification process described above, resolvin D5 produced from docosahexaenoic acid having a purity of 90% obtained by hydrolysis and subsequent solvent extraction exhibited a purification yield improved by 25.3% compared to a control group, while resolvin D5 produced from a docosahexaenoic acid reagent having a purity of 95% exhibited a purification yield improved by 23.1% compared to the control group (Table 2).

Table 2 below shows the purity, yield, and recovery rate at each step of the purification process of resolvin D5 produced in the bioreactor using the ultrafiltration, the C18 octadecyl resin, the preparative column chromatography, and the adsorption resin.

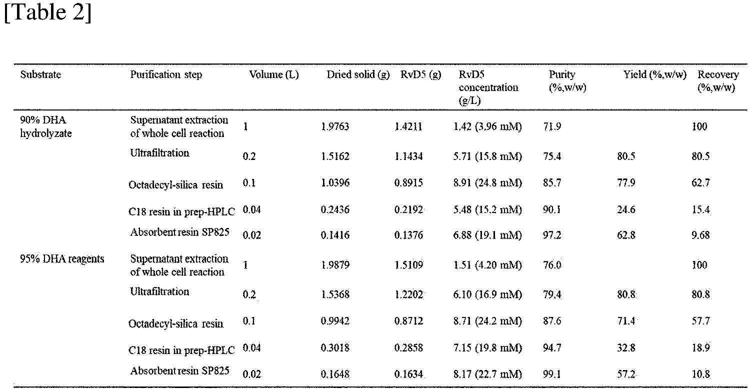

As described above, the present invention relates to a method in which an oil obtained from fish or microalgae is hydrolyzed into polyunsaturated fatty acids comprising docosahexaenoic acid using a commercial lipase and an adsorption resin, and through subsequent solvent extraction, a docosahexaenoic acid substrate having a purity of 90% was secured. Using, as substrates, the obtained docosahexaenoic acid having a purity of 90%, as well as docosahexaenoic acid reagents having purities of 95% or 99%, reaction conditions for conversion into resolvin D5, an endogenous substance in the human body, were optimized in flasks and bioreactors through whole-cell reactions using a double arachidonate 15-lipoxygenase derived from the bacterium *Archangium violaceum.* As a result, the present invention provides a method for producing resolvin D5 at a high yield of 70% or more and a purification yield of 97% or more, at a g/L level, by means of a bioconversion process. Specifically, the substrates used include a docosahexaenoic acid-enriched oil, docosahexaenoic acid reagents having purities of 95% and 99%, and a hydrolysate comprising docosahexaenoic acid at a purity of 90%, and transformed cells comprising a double arachidonate 15-lipoxygenase derived from *Archangium violaceum* are employed. In a reaction mixture to which dimethyl sulfoxide as a solvent and polyvinylpyrrolidone as a polymer are added, resolvin D5 can be produced at an enhanced concentration through a bioconversion process in flasks and bioreactors, thereby enabling large-scale production. Accordingly, the present invention not only overcomes the limitations of conventional chemical synthesis methods under environmentally friendly conditions, but also has significant meaning in that it enables a unique large-scale production of an endogenous human substance by a bioconversion method that has not been previously reported.

Although specific portions of the present invention have been described in detail above, it will be apparent to those having ordinary skill in the art of fatty acids and pharmaceuticals that such detailed descriptions are merely exemplary embodiments and do not limit the scope of the present invention. Accordingly, the substantial scope of the present invention is defined by the appended claims and their equivalents.

### SEQUENCE LISTING

SEQ ID NO: 1
   15-lipoxygenase derived from *Archangium violaceum* strain 
MRSIPSLPQN DSPAQQELRL ATLTQERQVY AYNFDSRLSP LGIAAQVPRQ
DNFSFVWLDG
VATTGLQLLG NVLAIGAKLF DNGEAIQNDA GLSELELRRV EGTYRELTDG
FSRLSDRTSR
LHQAPRSPAV TLIADVIERP RTLLKAAGQK LEETAGVAIV SELATQAALD
 
APAIGQDILD
LIDTLLKKLL SEAGDLFLKY LGLYGKAASL DNYTAQFSLL QPPSVASNYE
TDLIFARMRL
AGPNPALLQG IAALPEKFPV TDAQYQSVMG SQDTLARAGQ EGRLYLLDYA
VFQGIPTGQT
SGGQKYIEAP LALFAVPAAN QADRKLRAVA IQCSQTPGRS NPIFTPADGT
SWSLARLHVQ
VADGNYHELI AHLGLTHLIL EEFTLSTHRQ LAPQHPLYLL LTPHFQGTLA
INNAAETSLI
APGGPVDQLL AGEIKASTQV SIQAVANQSI NQSFLPRALA ARGVDDASKL
PDYPYRDDAL
LLWNDIRAWV SDYLAIYYND DAAVRSDYEL QAWVTELASP DAGKLKDVTE
GGGGIQTFEY
LVDLTTYIIF TASVQHAAVN FPQRTVMSYT PALPLAAYAP APTSVEELPP
STELTHLPPL
QMAFLQQAVT FGLGNVYFTR LGGYDTYLRE PWFADARVWP ALEVFQKRLR
ATEREIGQRN
LSRIPYETLL PTAIPQSINI Z
SEQ ID NO: 2

15-lipoxygenase derived from *Archangium violaceum* strain

atgcgttcca ttccctccct gccccagaat gactctcccg cccagcagga gctgcgcctc

gccacgctca cacaagagcg gcaggtctac gcctacaact tcgacagccg gctgagtccg
ctggggatcg cggcccaggt cccccgtcag gacaacttct ccttcgtctg gttggatggc
gtcgccacca ccggcctgca gctgctgggc aacgtgctgg ccatcggcgc gaagctcttc
gacaacgggg aggcgatcca gaacgacgcc ggcctgtccg agctggagct ccgcagggtc
gaggggacct accgggagct gaccgacgga ttcagccggc tgtcggaccg gacctcccgg
ctccaccagg cgccgcgctc cccggcggtg acgctcatcg ccgatgtcat cgagcgcccc
cggacgctgc tcaaggcggc gggccagaag ctggaggaga ccgccggtgt cgccatcgtc
tccgagctcg ccacccaggc cgcgctggat gcgcccgcca tcgggcagga catcctcgac
ctcatcgaca ccctgttgaa gaagctgttg tccgaggccg gagatctgtt cctgaagtac
ctggggctgt acggcaaggc ggcgtcgctc gacaactaca cggcccagtt ctcgctgctg
cagcctccgt cggtcgccag caattacgag accgacctca tcttcgcgcg catgcggctg
gccgggccca acccggcgct gctccagggc atcgcggccc tccccgagaa gttcccggtg
acggacgcgc agtaccagtc cgtgatgggc tcgcaggaca ccctggcccg cgcgggccag
gaaggtcggc tgtacctgct ggactacgcc gtcttccagg gcatccccac cggacagacg
tcgggcggac agaagtacat cgaagcgccg ctcgccctgt tcgccgtgcc cgccgcgaac
caggccgacc ggaagctgcg cgcggtggcc atccagtgct cgcagacgcc gggccgctcc
aaccccatct tcaccccggc ggacggcacc tcctggagcc tggcgcgcct gcacgtgcag
gtggcggatg gcaactacca cgagctcatc gcgcacctgg gcctcacgca cctgattctg
gaagagttca ccctgtccac gcaccggcag ctcgcgcccc agcacccgct ctacctgctg
ctgacgccgc acttccaggg caccctggcc atcaacaacg ccgcggagac gtccctcatc
gcgcccggtg gcccggtgga ccagctgctc gccggtgaga tcaaggcctc cacccaggtg
agcatccagg ccgtggccaa ccaatccatc aaccagtcgt tcctgccccg ggcgctggcg

gcccgcgggg tggacgacgc ctcgaagctg ccggactacc cgtaccgcga cgacgcgctg
ctgctctgga acgacatccg cgcctgggtg agcgactacc tggccatcta ctacaacgat
gacgcggcgg tgcggagcga ctacgagctg caggcctggg tcacggagct cgccagcccc
gacgccggca agctgaagga tgtgacggag ggcggagggg gcatccagac cttcgagtat
ctggtggacc tgaccaccta catcatcttc acggcgagcg tgcagcacgc ggcggtcaac
ttcccgcagc gcacggtcat gagctacacc ccggcgctgc cgctggcggc ctacgctccc
gcgcccacct ccgtggaaga gctgccgccg agcacggagc tgacccacct gccgccgttg
cagatggcct tcctgcagca ggcggtcacc ttcgggctgg gcaacgtgta cttcacccgg
ctgggcggct acgacacgta cctgcgcgag ccctggttcg cggacgcgcg cgtgtggccc
gccctggagg tcttccagaa gcgcctgcgc gccacggagc gggagatcgg ccagcgcaac
ctgtcgcgca tcccctacga gacgctgctg cccacggcga ttccgcagag catcaacatc
taa

## Claims

1. A composition comprising a polyunsaturated fatty acid at a high purity derived from a DHA-containing oil obtained from fish or microalgae, for use in producing a dihydroxylated fatty acid having 20 to 22 carbon atoms.

2. The composition of claim 1,
wherein the polyunsaturated fatty acid is obtained by hydrolyzing the DHA-containing oil obtained from fish or microalgae.

3. The composition of claim 2,
wherein the hydrolysate of the DHA-containing oil obtained from fish or microalgae is obtained by hydrolyzing the DHA-containing oil using a lipase and an adsorption resin.

4. The composition of claim 3,
wherein the lipase is a lipase derived from *Thermomyces lanuginosus,* and the adsorption resin is SP207, HP20, HP2MG, SP825, SP850, or XAD761.

5. The composition of claim 2,
wherein the polyunsaturated fatty acid is obtained by purifying the hydrolysate of the DHA-containing oil obtained from fish or microalgae with a solvent in which hexane and acetonitrile are mixed at a volume ratio of 3 to 5:1.

6. The composition of claim 1,
wherein the polyunsaturated fatty acid comprises at least one fatty acid selected from the group consisting of docosahexaenoic acid, eicosapentaenoic acid, docosapentaenoic acid, and arachidonic acid.

7. The composition of claim 1,
wherein the dihydroxylated fatty acid having 20 to 22 carbon atoms comprises resolvin D5.

8. The composition of claim 1,
wherein the composition comprises docosahexaenoic acid as the polyunsaturated fatty acid at a high purity of 70 to 99%.

9. A method for producing resolvin D5, comprising:
a) preparing a vector encoding a double 15-lipoxygenase gene derived from an *Archangium violaceum;*
b) transforming a microorganism with the vector of step a); and
c) reacting the transformed microorganism of step b) with a composition comprising a polyunsaturated fatty acid at a high purity derived from a DHA-containing oil obtained from fish or microalgae as a substrate, thereby producing resolvin D5.

10. The method of claim 9,
further comprising, after step c), a step d) of purification for producing resolvin D5 at a high yield.

11. The method of claim 10,
wherein the purification step d) is performed simultaneously or sequentially by ultrafiltration, column filtration, affinity chromatography, and reversed-phase chromatography.

12. The method of claim 10,
wherein the affinity chromatography is performed using an adsorption resin selected from HP20, HP2MG, SP207, SP825, SP850, or XAD761.

13. The method of claim 10,
wherein the reversed-phase chromatography is performed using a C1 (TMS) resin, a C4 (butyl) resin, a C8 (octyl) resin, or a C18 (octadecyl) resin.

14. The method of claim 9,
wherein the polyunsaturated fatty acid comprises at least one fatty acid selected from the group consisting of docosahexaenoic acid, eicosapentaenoic acid, docosapentaenoic acid, and arachidonic acid.

15. The method of claim 9,
wherein the composition of step c) comprises docosahexaenoic acid as the polyunsaturated fatty acid at a high purity of 70 to 99%, and is obtained by hydrolyzing the DHA-containing oil using a lipase and an adsorption resin.

16. The method of claim 9,
wherein the double 15-lipoxygenase of step a) comprises an amino acid sequence of SEQ ID NO: 1 or a sequence having at least 80% sequence identity to SEQ ID NO: 1.

17. The method of claim 15,
wherein the polyunsaturated fatty acid is obtained by purifying a hydrolysate of the DHA-containing oil obtained from fish or microalgae with a solvent in which hexane and acetonitrile are mixed at a volume ratio of 3 to 5:1.

18. The method of claim 15,
wherein, in the hydrolysis step, when the concentration of the DHA-containing oil obtained from fish or microalgae is 5 to 15 g/L, the lipase is treated at a concentration of 800 to 1200 U/mL.

19. The method of claim 9,
wherein the reaction is performed at a pH in a range of 6 to 9.

20. The method of claim 9,
wherein the reaction is performed at a temperature in a range of 10 to 40 °C.

21. The method of claim 9,
wherein, in the step c), a solvent and a polymer are added to the reaction mixture to increase the solubility of the substrate.

22. The method of claim 21,
wherein the solvent comprises at least one selected from the group consisting of isopropyl alcohol (IPA), methanol (MeOH), ethanol (EtOH), ethyl acetate (EA), and dimethyl sulfoxide (DMSO).

23. The method of claim 22,
wherein the concentration of dimethyl sulfoxide (DMSO) included in the solvent is 0.1% to 10%.

24. The method of claim 21,
wherein the polymer comprises at least one selected from the group consisting of polyvinylpyrrolidone (PVP), polyvinyl alcohol (PVA), and polyethylene glycol (PEG).

25. The method of claim 24,
wherein the polymer is polyvinylpyrrolidone (PVP), and the concentration of the polyvinylpyrrolidone applied is 0.1% to 10%.
